# EUROPEAN PATENT APPLICATION

(11) **EP 0 543 406 A1**
(43) Date of publication of application: **26.05.1993**
(21) Application number: 92119823.0
(22) Date of filing: 20.11.1992
(51) Int. Cl.: A61M 35/00, A45D 40/00

(54) **Package containing a single dose of a substance**

(30) Priority: 22.11.1991 IL 100133
(71) Applicant: Meir, Jacobsohn, Tel-Aviv (IL); Ben-Zion, Klein, Hertzlia (IL)
(72) Inventor: Meir, Jacobsohn, Tel-Aviv (IL); Ben-Zion, Klein, Hertzlia (IL)
(74) Representative: VOSSIUS & PARTNER

(57) **Abstract**

A package for dosed substances, such as pharmaceuticals, therapeutical, cosmetic and sanitary substances comprises a container of the type of a sac, envelope or two hingedly connecting parts (1,5) adapted to be folded onto each other. The container holds a dose (4) of an active substance of the above mentioned kind which - when liquid - is soaked in a piece of cotton wool or gauze and when a solid is directly included in the container.

## Description

### BACKGROUND AND FIELD OF INVENTION

This invention concerns a package containing a one time, single dose of a substance, such as a pharmaceutical, therapeutical, cosmetic or sanitary preparations.

### OBJECT OF THE INVENTION

It is the object of the invention to present and to permit application of the said substance to the human body in a manner which precludes the contact thereof with human hands or other bodies which might contaminate the substance.

It is yet a further object of the present invention to provide a package which enables the use thereof without contact of the user's hands avoiding the washing of the hands if the nature of the substance so requires.

It is a further object of the invention to provide a dosed quantity of the active substance contained in the package.

It is also an object of the invention to provide a sterile, compact package of the above mentioned substances.

### SHORT SUMMARY OF THE INVENTION

According to the invention the new package comprises basically, in a simple form, a sac or small envelope like containing means consisting of two sheets connected at their circumferential edges and between the two sheets a dose of the active substance which is contained, if liquid, imbued in a carrier of the type of cotton wool, a small piece of gauze or textile fabric or any other matter which may be impregnated with liquid. Solid substances, such as pharmaceuticals in the form of creams, ointments, etc. may be included in the sac like container or envelope being directly attached to one of the sheets forming the sac or envelope.

In a practical embodiment the package comprises a container consisting of two hingedly connected concave parts which are adapted to be folded onto one another and hold the respective substance in the cavity formed by the two parts when these are folded onto each other.

The invention will now be described in detail with reference to the annexed drawings, in which:
Fig. 1 is a schematical plan view of one of the sheets constituting the sac or envelope like container.
Fig. 2 shows the same sheet with a pad or membrane carrying the above substance attached thereto.
Fig. 3 shows the second sheet forming the sac with the one of Fig. 1.
Fig. 4 illustrates schematically the complete package and its contents.
Fig. 5 is a sectional view of the new package.

A practical embodiment of the new package is illustrated by Figs. 6 - 8.
Fig. 6 being a plan view of the package, in open position.
Fig. 7 shows the package in closed state, while
Fig. 8 is a like view of a slightly varied formation.

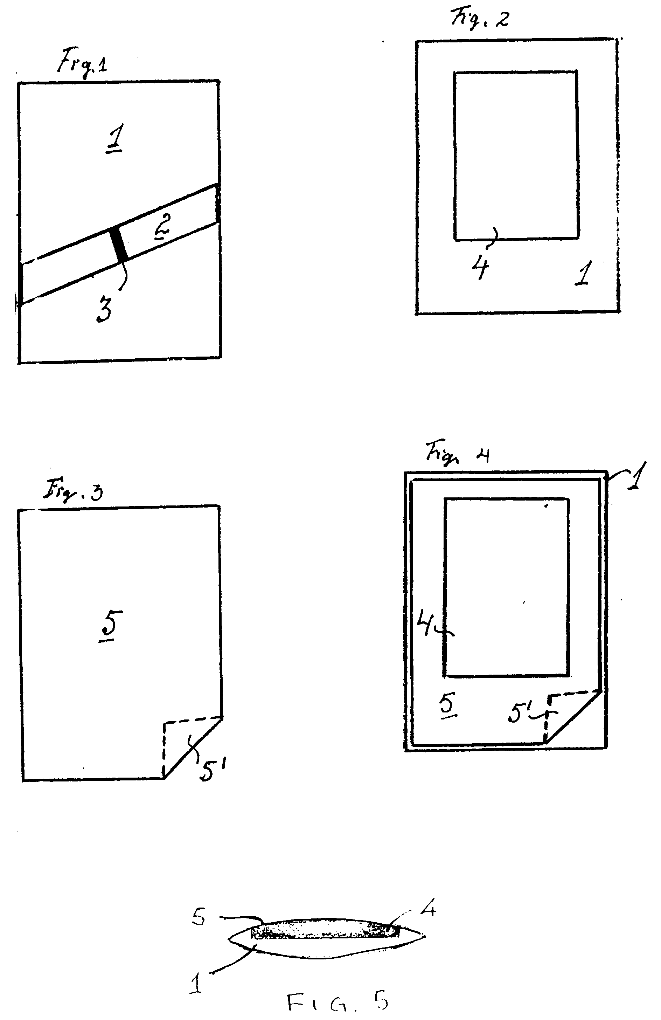

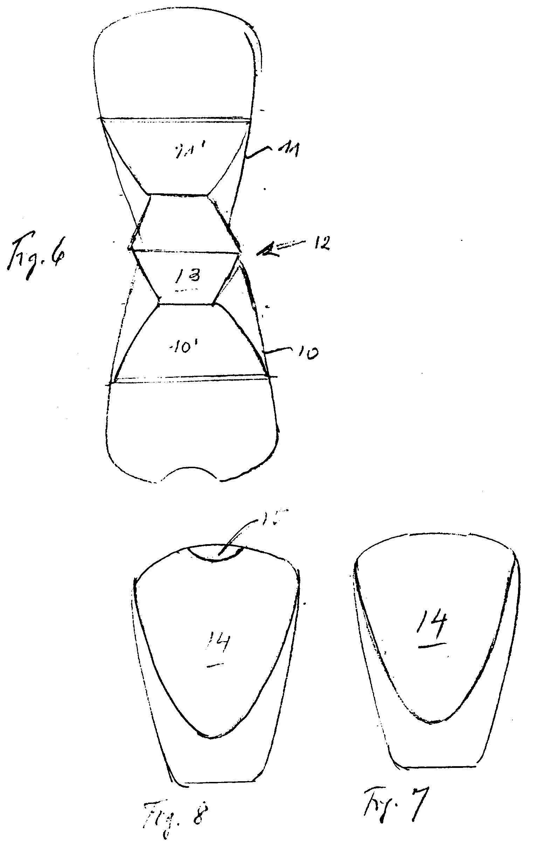

Turning first to Fig. 1, there is shown a preferably moderately stiff sheet 1 of cardboard or strong paper or any other such or similar material to one side of which is affixed an obliquely across extending strip 2 of paper or plastics attached to sheet 1 at two opposite edges of the latter. This strip forms a hoop through which a person can pass one or more fingers holding the package. An out-jutting ridge 3 is provided permitting to grasp the package and transfer it from one hand to the other hand of a person without coming in direct contact with the substance thereof. The reverse side of sheet 1 is shown in Fig. 2. To that side of sheet 1 is affixed e.g. a piece 4 of gauze which is impregnated with a liquid, say an after shave lotion or a substance such as cream. To the same sheet 1 is attached a sheet 5 being vacuum sealed thereto at all four edges of the two sheets, except at a small triangular folded over corner piece 5'.

In the operation of preparing the package the piece of cotton wool or gauze or the like is affixed by an adhestive or otherwise to the sheet 1. In a further step the sheet 5 which may be a transparent foil or membrane is heat sealed under vacuum to sheet 1, leaving the folded over corner piece 5' unattached to sheet 1. The package with its contents of pharmaceutical, therapeutical, cosmetic or other contents may now reach a consumer via the customary channels of distribution. The consumer enters one finger or more into hoop 2 then detaches foil 5 by pulling it off with the aid of corner piece 5' and has now at her or his disposal the impregnated gauze or other matter 4 which carries the active substance and can make use of it in the customary way by holding the slightly stiff sheet 1, without touching piece 4.

The preparation, i.e. assembly of the package and its contents is carried out by known and customarily employed devices and implements without human hands touching the active substance. After having applied the substance to the user's face or other body parts, the sheet 1 with the piece of gauze or cotton wool adhering thereto can be committed to a refuse container.

It will be seen that by the novel means an active substance can be supplied to a consumer and can be used by the latter in a strictly hygienic way without any likelihood of contamination.

The practical embodiments of Figs. 6-8, two shell shaped rigid parts 10 and 11 which are either integrally moulded in one piece and are foldable about a line 12 or are connected by hinged means at that line 12.

Parts 10 and 11 have each a depression which in the closed state of the package register and together form a cavity. To the two parts is attached a foldable piece 13 of gauze or other textile or fibrous material. The outwardly bulging portion caused by the inner recess is designated in Figs. 7 and 8 by the numeral 14. The variant shown in Fig. 8 is fully identical with the described items of Figs. 6 and 7, but has a small cut-out 15 at the edge of one of the two parts 10,11 which bow shaped cut-out 15 facilitates the opening of the package.

While in the above description of both embodiments, the package has been described as containing a liquid substance, it should be clear that in a similar way a solid material could be included in the package by directly attaching it to one of the sheets constituting the sac or envelope of the first embodiment or could be placed in the cavity formed in the embodiment of Figs. 6-8.

## Claims

1. Package including a one time, single dose of a substance, such as a pharmaceutical, therapeutical, cosmetic or sanitary preparation, characterised thereby that it comprises a sac or small envelope like containing means consisting of two parts connected to each other between the two parts a dose of the active substance which is contained, if liquid, imbued in a carrier of the type of cotton wool, a small piece of gauze or textile fabric or any other matter which may be impregnated with liquid.

2. The package according to claim 1, characterised thereby that the said substance is solid or semi solid and is included in the space between the two parts being directly attached to one of the parts forming the package.

3. Package according to claim 1 wherein the two parts are shell shaped and rigid, each having a depression which two depressions are in register when the two parts are closed onto one another and form a cavity adapted to hold a substance as stated in the description.

4. Package according to claim 3, characterised thereby that a foldable piece of textile or fibrous material is attached to both parts.
